(19) Europäisches Patentamt

European Patent Office

Office européen des brevets

(11) **EP 4 497 462 A1**

(12) **EUROPEAN PATENT APPLICATION**

(43) Date of publication:
**29.01.2025 Bulletin 2025/05**

(21) Application number: **24190017.4**

(22) Date of filing: **22.07.2024**

(51) International Patent Classification (IPC):
**A61M 16/04** *(2006.01)*

(52) Cooperative Patent Classification (CPC):
**A61M 16/0431; A61M 16/0493; A61M 16/0495; A61M 16/0415**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **25.07.2023 TW 112127822**

(71) Applicant: **Chen, Tien-Sheng**
**Taipei City 112 (TW)**

(72) Inventor: **Chen, Tien-Sheng**
**Taipei City 112 (TW)**

(74) Representative: **Viering, Jentschura & Partner mbB**
**Patent- und Rechtsanwälte**
**Am Brauhaus 8**
**01099 Dresden (DE)**

(54) **OROPHARYNGEAL AIRWAY**

(57)  An oropharyngeal airway(1, 1a) has a plurality of fenestrations(10, 10a, 10b) and a curved tube(20), wherein the curved tube(20) has a curved front section(22) including a front end(221), the plurality of fenestrations(10, 10a, 10b) is disposed in the curved front section(22), the curved tube(20) has a curved tube length D1, and the curved front section(20) has a front length D2, where

$$D2 \leq \frac{1}{3} D1$$

.

FIG. 3

**Description**

BACKGROUND OF THE INVENTION

Field of the Invention

The present invention relates to an oropharyngeal airway, and more particularly, to an oropharyngeal airway that can establish a continuous breathing passage regardless of the length thereof.

Description of the Related Art

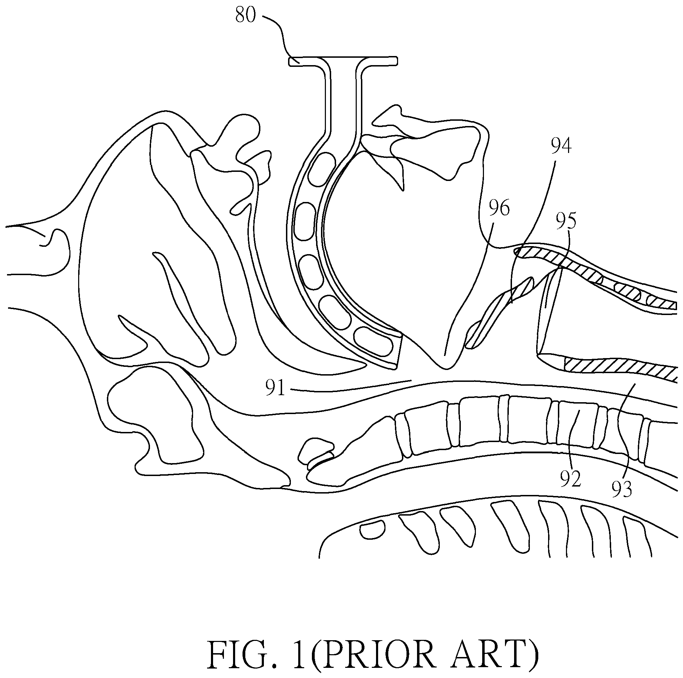

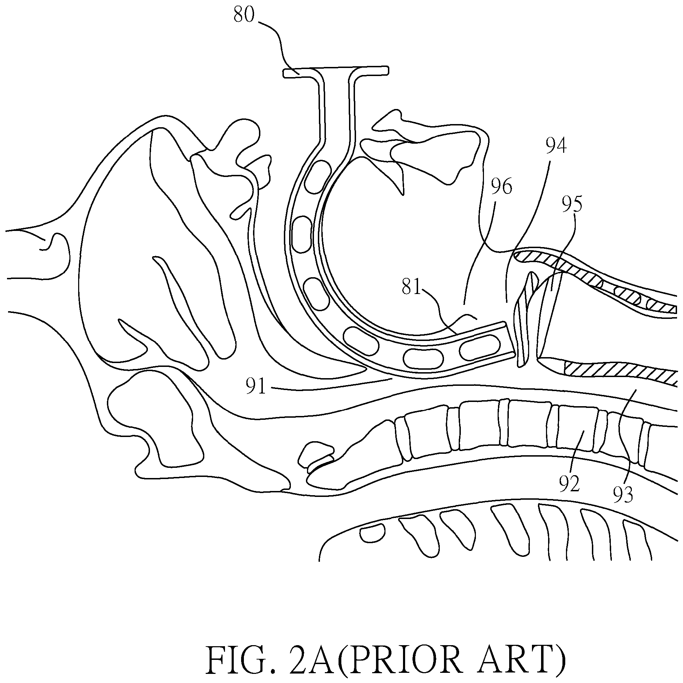

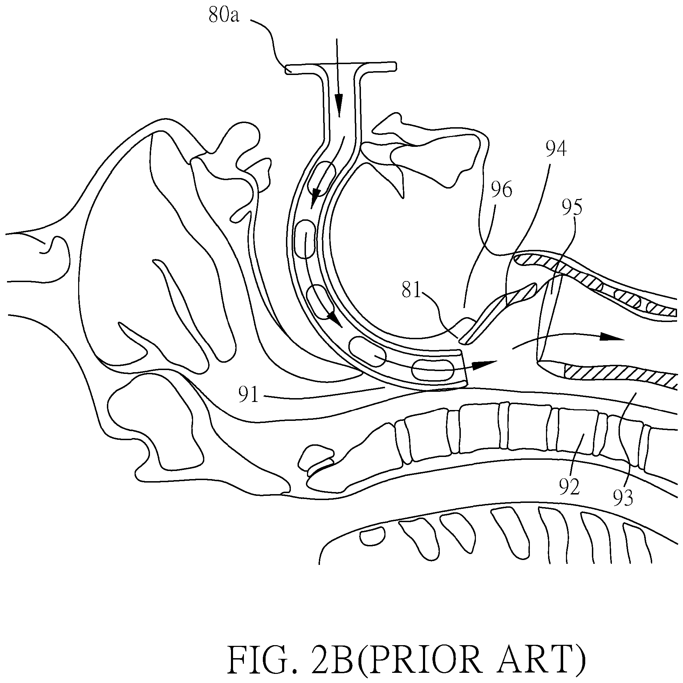

Please refer to FIG. 1, FIG. 2A, and FIG. 2B for a schematic view of a prior art oropharyngeal airway of insufficient length being placed in the pharynx; a schematic view of a prior art oropharyngeal airway of excessive length being placed in the pharynx; and a schematic view of a prior art oropharyngeal airway placed in the pharynx in a standard use condition. The oropharyngeal airway is a device used to lift a patient's tongue base to prevent the tongue from blocking the patient's respiratory tract. Two types of oropharyngeal airways commonly in use at present. One, called a Berman tube, is the oropharyngeal airway 80 shown in FIG. 1 and FIG. 2A. The Berman airway comprises two parallel curved pieces and a vertical connecting piece, wherein the two ends of the long axis of the vertical connecting piece are respectively connected to the two curved pieces. The other type is the Guedel airway. This type of oropharyngeal airway is directly formed as a curved hollow tube. The problems it presents are the same as those of the Berman airway, so no details or drawings of the Guedel airway will be presented herein.

As shown in FIG. 1 and FIG. 2A, when the patient loses consciousness or is in a coma, the tongue base 96 (when the patient is lying flat) falls due to gravity and blocks the tracheal opening 95. At this time, a healthcare worker will estimate the length from the patient's mouth to the throat based on the patient's profile, select an oropharyngeal airway 80 that may be of suitable length, and insert it into the patient's pharynx, thereby causing the oropharyngeal airway 80 to lift the tongue base 96 and to establish a continuous breathing passage from the mouth, the pharynx 91, past the tongue base 96, and to the tracheal opening 95 to maintain the patient's breathing, forming a state shown in FIG. 2B. However, this method is only for reference. In actual operation, due to individual differences in body structure, the states shown in FIG. 1 and FIG. 2 will occur, resulting in the problem that the patient's breathing passage cannot be established.

As shown in FIG. 1, when the oropharyngeal airway 80 inserted into the patient's pharynx is of insufficient length, the oropharyngeal airway 80 cannot fully support the tongue base 96, resulting in part of the tongue base 96 not being lifted by the oropharyngeal airway 80 and continuing to block the tracheal opening 95 such that a continuous breathing passage from the mouth, the pharynx 91, past the tongue base 96, and to the tracheal opening 95 cannot be established. At this time, a healthcare worker needs to remove the inappropriate oropharyngeal airway 80 and then select an oropharyngeal airway 80 of a different length to be inserted into the patient's pharynx. In contrast, when the oropharyngeal airway 80 inserted into the patient's pharynx is of excessive length, the front end will enter the esophagus and the air will flow along the oropharyngeal airway from the outside of the mouth into the esophagus without entering the trachea. If the length is appropriate but the patient's epiglottis is slightly longer, as shown in FIG. 2A, the front end 81 of the oropharyngeal airway 80 will press against the epiglottis 94, causing the epiglottis 94 to cover the tracheal opening 95 such that a continuous breathing passage cannot be established. Regarding the states shown in FIG. 1 or FIG. 2A, a healthcare worker will need to remove the inappropriate oropharyngeal airway 80 and then select an oropharyngeal airway 80 of a different length to be inserted into the patient's pharynx. Taking the widely used oropharyngeal airway as an example, a healthcare worker often needs to try to insert a few oropharyngeal airways 80 of different lengths into a patient's pharynx before finding the right one for the patient. In cases where a patient's epiglottis is too long, it is very likely that the healthcare worker will be unable to find an appropriate oropharyngeal airway 80, so there is a need for improvement.

SUMMARY OF THE INVENTION

The main object of the present invention is to provide an oropharyngeal airway that can establish a continuous breathing passage regardless of the length thereof.

In order to achieve the above object, the present invention provides an oropharyngeal airway comprising a plurality of fenestrations and a curved tube, wherein the curved tube includes a curved front section including a front end, the plurality of fenestrations is disposed in the curved front section, the curved tube has a curved tube length D1, and the curved front section has a front length D2, where $D2 \leq \frac{1}{3}D1$.

The curved front section of the oropharyngeal airway of the present invention is provided with an elongated fenestration. When the oropharyngeal airway is placed in the pharynx, the front end of the oropharyngeal airway passes beyond the tongue base and the epiglottis and enters the esophagus such that the oropharyngeal airway of the present invention lifts the patient's tongue base, pushes up the epiglottis, and establishes a breathing passage with a plurality of fenestrations, so that the healthcare worker does not need to worry that the oropharyngeal airway is of an inappropriate length or that a continuous breathing passage may not be established. It also solves the problem in the prior art that a healthcare worker must insert oropharyngeal airways of different lengths into the patient's pharynx before finding the appropriate oropharyngeal airway for the patient.

## BRIEF DESCRIPTION OF THE DRAWINGS

FIG. 1 illustrates a schematic view of a prior art oropharyngeal airway of insufficient length placed in the pharynx;

FIG. 2A illustrates a schematic view of a prior art oropharyngeal airway of excessive length placed in the pharynx;

FIG. 2B illustrates a schematic view of a prior art oropharyngeal airway placed in the pharynx in a standard use condition;

FIG. 3 illustrates a perspective view of a first embodiment of an oropharyngeal airway of the present invention;

FIG. 4 illustrates a bottom view of the first embodiment of the oropharyngeal airway of the present invention;

FIG. 5 illustrates a cross-sectional view of the first embodiment of the oropharyngeal airway inserted into the pharynx;

FIG. 6 illustrates a perspective view of a second embodiment of the oropharyngeal airway of the present invention;

FIG. 7 illustrates a bottom view and a partially cross-sectional view along the line A-A of the second embodiment of the oropharyngeal airway of the present invention; and

FIG. 8 illustrates a cross-sectional view of the second embodiment of the oropharyngeal airway of the present invention inserted into the pharynx.

## DETAILED DESCRIPTION OF THE EMBODIMENTS

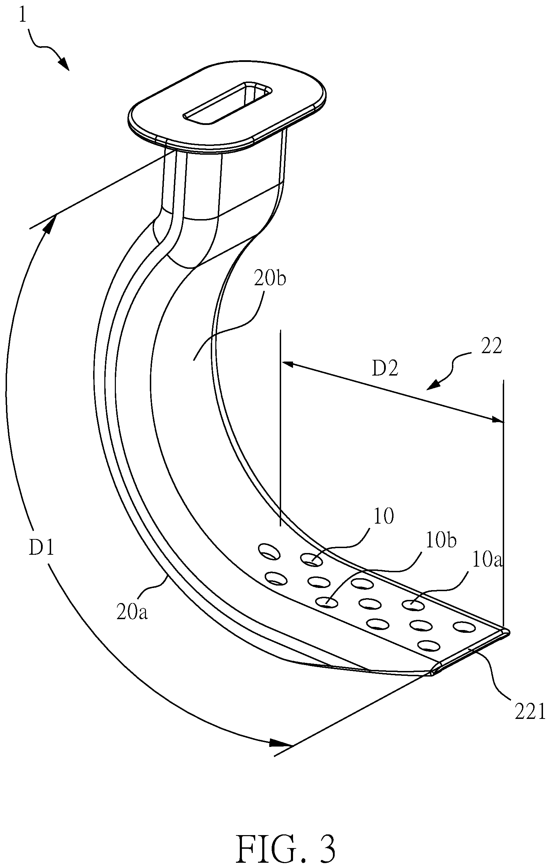

In order to better understand the technical content of the present invention, preferred embodiments are described as follows. Please refer to FIG. 3 to FIG. 5 for a perspective view and bottom view of a first embodiment of an oropharyngeal airway of the present invention and for a cross-sectional view of the oropharyngeal airway inserted into the pharynx.

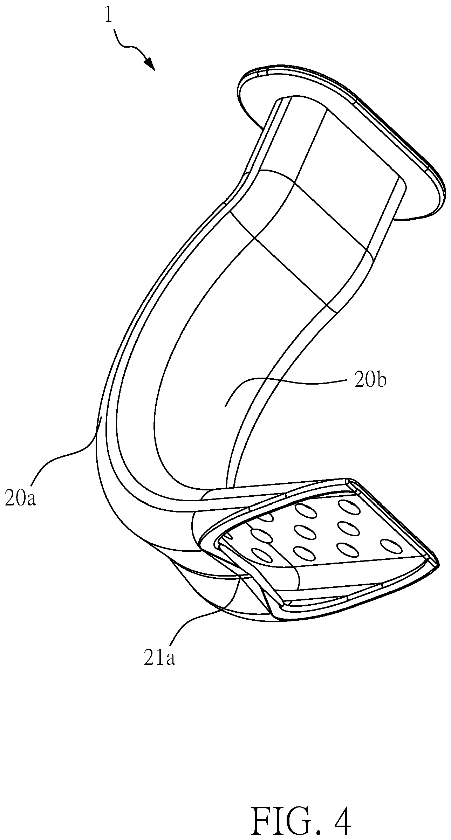

As shown in FIG. 3 and FIG. 4, the oropharyngeal airway 1 of this embodiment is a curved hollow tube body and includes a plurality of fenestrations 10, 10a, 10b and a curved tube 20, wherein the curved tube 20 is a curved hollow tube body. In this embodiment, the curved tube 20 includes a curved front section 22, and the curved front section 22 includes a front end 221, wherein the fenestrations 10, 10a, 10b are disposed in the curved front section 22; the curved tube 20 also has a curved tube length D1 and the curved front section 22 has a front section length D2, where $D2 \le \frac{1}{3} D1$. According to a preferred embodiment of the present invention, the curved tube length D1 can be 12 cm < D1 < 18 cm or 14 cm < D1 < 18 cm, and the front section length D2 may be 2 cm < D2 < 6 cm or 3 cm < D2 < 6 cm. However, the curved tube length D1 and the front section length D2 of the present invention are not limited to the aforementioned length ranges, as long as the condition that

$D2 \le \frac{1}{3}$ D1 is met. As shown in FIG. 3, in this embodiment, the curved tube length D1 is the length of the curved hollow tube, and the front section length D2 refers to the distance from the front end 221 to the farthest fenestration 10. In this embodiment, the plurality of fenestrations 10, 10a, and 10b is a plurality of ventilation holes.

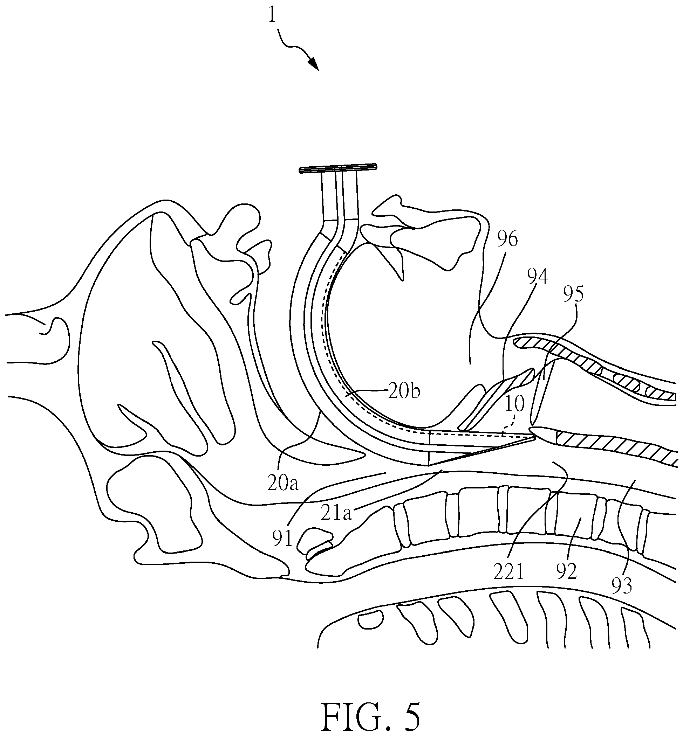

As shown in FIG. 3 and FIG. 4, the curved tube 20 has a curved inner side 20a and a curved outer side 20b, wherein the fenestrations 10, 10a, 10b are disposed only on the curved outer side 20b, the fenestrations 10, 10a, 10b are dispersed from the front end 221 to the curved outer side 20b, and the curved inner side 20a includes a groove 21a. As shown in FIG. 5, when the oropharyngeal airway 1 of the present invention enters the pharynx 91 through the human mouth, the curved outer side 20b of the oropharyngeal airway 1 is close to one side of the tracheal opening 95, the curved inner side 20a of the oropharyngeal airway 1 is close to one side of the cervical vertebra 92, the groove 21a is in contact with the cervical vertebra 92, and the concave shape of the curved inner side 20a formed by the groove 21a allows the oropharyngeal airway 1 to be firmly placed on the cervical vertebra 92 to prevent the oropharyngeal airway 1 from moving.

The object of the groove 21a is now explained in detail. Since the cervical vertebra 92 is formed in an arc shape, if the groove 21a is not provided, the smooth surface of the curved inner side 20a will be in direct contact with the cervical vertebra 92, and the arc shape of the vertebra 92 will not provide stable support for the smooth surface of the curved inner side 20a, causing the oropharyngeal airway 1 located above the cervical vertebra 92 to slide relative to the cervical vertebra 92 and resulting in displacement of the oropharyngeal airway 1.

As shown in FIG. 5, when the oropharyngeal airway 1 is placed in the pharynx 91, the front end 221 of the oropharyngeal airway 1 of the present invention enters the esophagus 93, and the fenestrations 10, 10a, 10b (ventilation holes in this embodiment) on the curved outer side 20b provide ventilation for the patient at the tracheal opening 95. In order to ensure an adequate amount of ventilation by the oropharyngeal airway 1 of the present invention, according to a preferred embodiment of the present invention, the area of the plurality of fenestrations 10, 10a, 10b accounts for 3% to 40%, 10% to 40%, or 10% to 30% of the area of the curved front section 22, or at least one fenestration (ventilation hole) and at most 5 fenestrations (ventilation holes) can be dispersed in a unit area of a square centimeter of the curved front section 22.

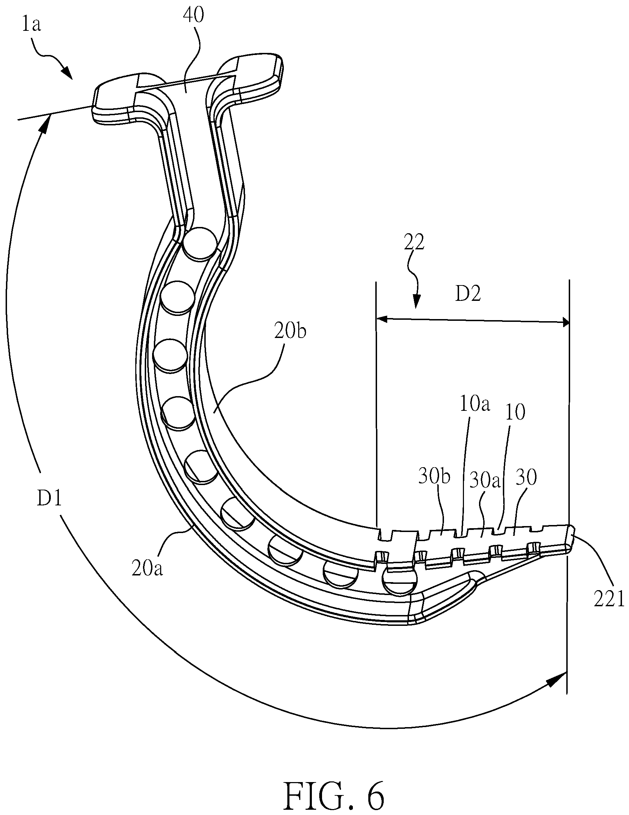

Please refer to FIG. 6 to FIG. 8 for a perspective view and a bottom view of an oropharyngeal airway of a second embodiment of the present invention and for a cross-sectional view of the oropharyngeal airway of the second embodiment entering the pharynx.

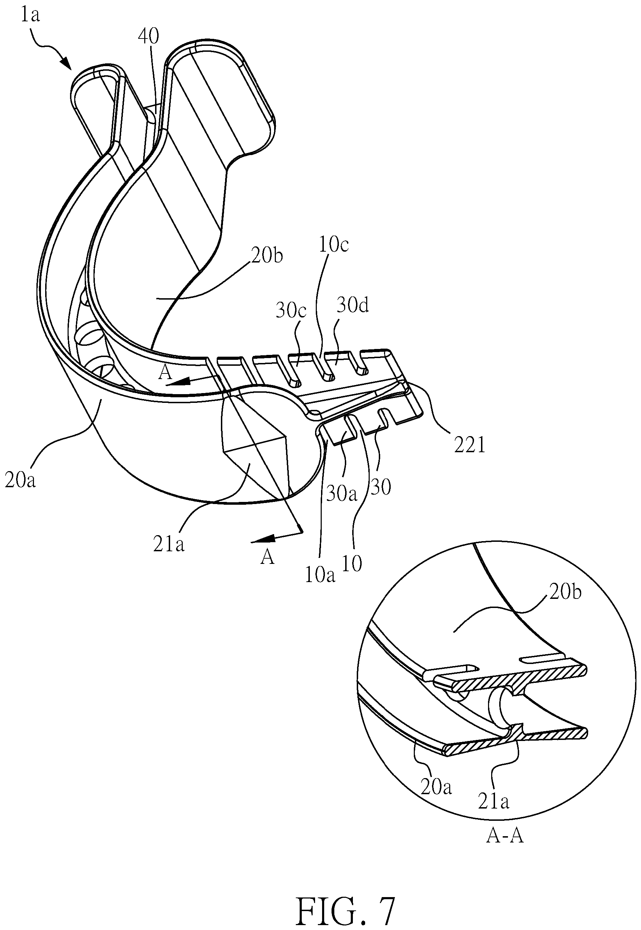

As shown in FIG. 6 and FIG. 7, the curved tube 20 of the oropharyngeal airway 1a of a second embodiment is comprised of two parallel curved pieces and a vertical connecting piece, wherein the two parallel curved pieces are respectively a curved outer side 20b and a curved inner side 20a, the curved outer side 20b and the curved inner side 20a are connected by a connecting piece 40, and the curved inner side 20a includes a groove 21a. In addition, the oropharyngeal airway 1a of the second embodiment further includes a plurality of splitter bars 30, 30a, 30b, 30c, 30d, wherein each splitter bar 30, 30a, 30b, 30c, 30d is disposed on the curved outer side 20b and the plurality of fenestrations 10, 10a, 10b, 10c of the oropharyngeal airway 1a of the second embodiment are distributed between two adjacent splitter bars 30, 30a, 30b, 30c, and 30d.

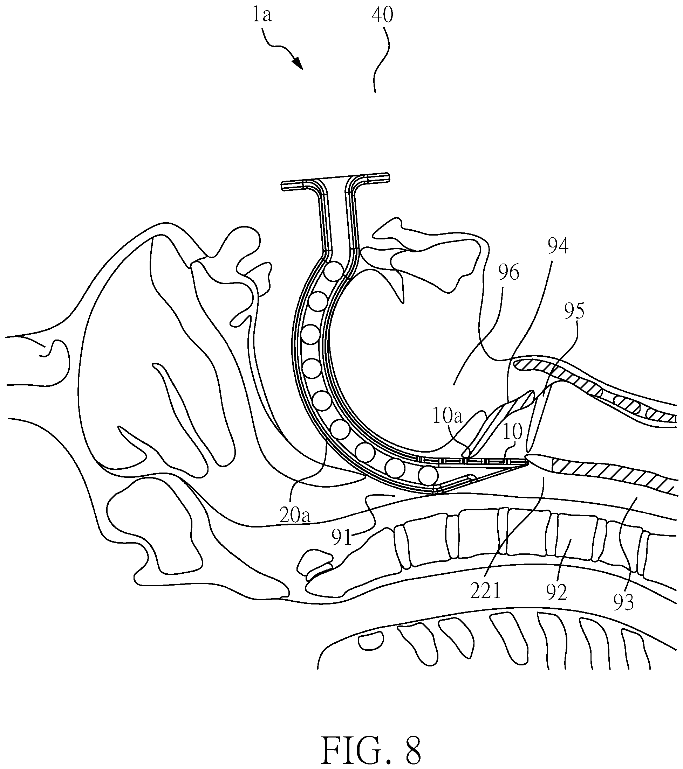

As shown in FIG. 8, when the oropharyngeal airway 1a of the second embodiment of the present invention enters the pharynx 91 through the human mouth, the curved outer side 20b of the oropharyngeal airway 1 is close to one side of the tracheal opening 95, the curved inner side 20a of the oropharyngeal airway 1 is close to one side of the cervical vertebra 92, the groove 21a is in contact with the cervical vertebra 92, and the concave shape of the curved inner side 20a formed by the groove 21a allows the oropharyngeal airway 1 to be firmly placed on the cervical vertebra 92 to prevent the oropharyngeal airway 1 from moving (for a detailed description of this part, please refer to the similar paragraphs of the first embodiment; no further description will be provided here). When the oropharyngeal airway 1a is placed in the pharynx 91, the front end 221 of the oropharyngeal airway 1a of the present invention enters the esophagus 93 and the fenestrations (which are the gaps between two adjacent splitter bars 30, 30a, 30b, 30c, 30d in this embodiment) 10, 10a, 10b of the curved outer side 20b provide oxygen to the patient at the tracheal opening 95. In order to ensure a sufficient amount of ventilation by the oropharyngeal airway 1 of the present invention, according to a preferred embodiment of the present invention, the area of the plurality of fenestrations 10, 10a, 10b accounts for 3% to 40%, 10% to 40%, or 10% to 30% of the area of the curved front section 22, or at least one fenestration (ventilation hole) and at most 3 to 5 fenestrations (ventilation holes) can be dispersed in a unit area of a square centimeter of the curved front section 22.

The curved front sections 22 of the oropharyngeal airways 1, 1a of the present invention are provided with fenestrations 10, 10a, 10b, 10c, 10d so that when the oropharyngeal airway 1, 1a is placed in the pharynx, the front end of the oropharyngeal airway 1, 1a passes beyond the tongue base and the epiglottis and enters the esophagus 93 such that the oropharyngeal airway 1, 1a of the present invention lifts the patient's tongue base 96 and establishes a breathing passage with a plurality of fenestrations; thus, the healthcare worker does not need to worry that the oropharyngeal airway 1, 1a is of an inappropriate length or that a continuous breathing passage may not be established. It also solves the problem of the prior art that a healthcare worker must insert multiple oropharyngeal airways of different lengths into the patient's pharynx 91 before finding the appropriate oropharyngeal airway for the patient.

It is noted that the above-mentioned embodiments are examples provided merely for description, and the scope of the present invention should be based on the scope of the claims, rather than being limited to the above-mentioned embodiments.

**Claims**

1. An oropharyngeal airway(1, 1a) comprising:

   a plurality of fenestrations(10, 10a, 10b); and
   a curved tube(20), the curved tube comprising a curved front section(22) including a front end (221), wherein the plurality of fenestrations(10, 10a, 10b) is disposed in the curved front section(22), the curved tube(20) has a curved tube length D1, and the curved front section(22) has a

   $$D2 \leq \tfrac{1}{3} D1$$

   front length D2, where                    .

2. The oropharyngeal airway(1, 1a) as claimed in Claim 1, wherein the curved tube (20) has a curved inner side(20a) and a curved outer side(20b); when the oropharyngeal airway(1, 1a) is placed in a pharynx(91), the plurality of fenestrations(10, 10a, 10b) is disposed on the curved outer side(20b).

3. The oropharyngeal airway as claimed in Claim 2, wherein the plurality of fenestrations(10, 10a, 10b) is a plurality of ventilation holes.

4. The oropharyngeal airway(1, 1a) as claimed in Claim 3, wherein the curved inner side(20a) includes a groove(21a).

5. The oropharyngeal airway as claimed in Claim 4, wherein, when the oropharyngeal airway(1, 1a) is placed in the pharynx(91), the curved inner side(20a) is close to one side of the cervical vertebra(92).

6. The oropharyngeal airway as claimed in Claim 5, wherein, when the oropharyngeal airway(1, 1a) is placed in the pharynx(91), the front end(221) enters the esophagus(93).

7. The oropharyngeal airway(1, 1a) as claimed in Claim 2 further comprising a plurality of splitter bars(30, 30a, 30b, 30c, 30d), each splitter bar(30, 30a, 30b, 30c, 30d) being disposed on the curved outer side(20b).

8. The oropharyngeal airway(1, 1a) as claimed in Claim 7, wherein each fenestration(10, 10a, 10b) is distributed between two adjacent splitter bars(30, 30a, 30b, 30c, 30d).

9. The oropharyngeal airway(1, 1a) as claimed in Claim 7, wherein the curved inner side(20a) includes a groove(21a).

10. The oropharyngeal airway(1, 1a) as claimed in Claim 9, wherein, when the oropharyngeal airway(1, 1a) is placed in the pharynx(91), the curved inner side(20a) is close to one side of the cervical vertebra(92).

11. The oropharyngeal airway(1, 1a) as claimed in Claim 10, wherein, when the oropharyngeal airway(10, 10a, 10b) is placed in a pharynx(91), the front end (221) enters the esophagus(93).

12. The oropharyngeal airway(1, 1a) as claimed in Claim 2, wherein the area of the plurality of fenestrations(10, 10a, 10b) accounts for 3% to 40%, 10% to 40%, or 10% to 30% of the area of the curved front section(22), or at least one fenestration(10, 10a, 10b) and at most 3 to 5 fenestrations(10, 10a, 10b) can be dispersed in a unit area of a square centimeter of the curved front section(22).

FIG. 1(PRIOR ART)

FIG. 2A(PRIOR ART)

FIG. 2B(PRIOR ART)

FIG. 3

FIG. 4

FIG. 5

FIG. 6

FIG. 7

FIG. 8

Europäisches Patentamt
European Patent Office
Office européen des brevets

# EUROPEAN SEARCH REPORT

**Application Number**

EP 24 19 0017

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2003/000534 A1 (ALFERY DAVID D [US]) 2 January 2003 (2003-01-02) * The whole document, especially Figs 4A-7A and paragraphs [0062, 0064, 0066] * | 1-12 | INV. A61M16/04 |
| X | GB 2 591 238 A (MARAT SINGATULLIN [GB]) 28 July 2021 (2021-07-28) * The whole document, especially Figs 2-8; page 3, lines 12-27; page 5, lines 9-22 * | 1-6,12 | |
| X | WO 2018/200063 A1 (WEDGE THERAPEUTICS LLC [US]) 1 November 2018 (2018-11-01) * The whole document, especially Figs 1F-1H, 2, 3C-4, 8, 12A; page 34, line 33 to page 35, line 16; page 41, lines 32 to page 42, line 13 * | 1-6,12 | |
| X | WO 2014/011932 A1 (FINANCIAL CONSULTANTS LLC [US]) 16 January 2014 (2014-01-16) * The whole document, especially Figs 6 and 7; paragraphs [0046, 0048]; pages 1-3, columns 4-6 * | 1-6,12 | TECHNICAL FIELDS SEARCHED (IPC) A61M |
| X | US 2022/233800 A1 (LONGO MARC [US]) 28 July 2022 (2022-07-28) * The whole document, especially paragraphs [0038, 0042-0045] * | 1-6,12 | |
| X | US 2003/131853 A1 (WALL WILLIAM H [US]) 17 July 2003 (2003-07-17) * The whole document, especially paragraphs [0029-0035] * | 1-6,12 | |
| X | US 2020/171255 A1 (XIAO JINFANG [CN] ET AL) 4 June 2020 (2020-06-04) * paragraphs [0056], [0068] * | 1-3 | |

-/--

The present search report has been drawn up for all claims

| Place of search | Date of completion of the search | Examiner |
|---|---|---|
| Munich | 15 November 2024 | Borowski, Aleksander |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

# EUROPEAN SEARCH REPORT

**Application Number**

EP 24 19 0017

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WO 80/00538 A1 (LUOMANEN R) 3 April 1980 (1980-04-03) * the whole document * | 1-12 | |

- - - - -

TECHNICAL FIELDS
SEARCHED        (IPC)

The present search report has been drawn up for all claims

| Place of search | Date of completion of the search | Examiner |
|---|---|---|
| Munich | 15 November 2024 | Borowski, Aleksander |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

................................................................................
& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 24 19 0017

This annex lists the patent family members relating to the patent documents cited in the above-mentioned European search report.
The members are as contained in the European Patent Office EDP file on
The European Patent Office is in no way liable for these particulars which are merely given for the purpose of information.

15-11-2024

| Patent document cited in search report | | Publication date | Patent family member(s) | | Publication date |
|---|---|---|---|---|---|
| US 2003000534 | A1 | 02-01-2003 | US | 6386199 B1 | 14-05-2002 |
| | | | US | 2003000534 A1 | 02-01-2003 |
| | | | US | 2004182384 A1 | 23-09-2004 |
| GB 2591238 | A | 28-07-2021 | NONE | | |
| WO 2018200063 | A1 | 01-11-2018 | US | 2021093818 A1 | 01-04-2021 |
| | | | WO | 2018200063 A1 | 01-11-2018 |
| WO 2014011932 | A1 | 16-01-2014 | US | 2014135641 A1 | 15-05-2014 |
| | | | US | 2017239431 A1 | 24-08-2017 |
| | | | WO | 2014011932 A1 | 16-01-2014 |
| US 2022233800 | A1 | 28-07-2022 | NONE | | |
| US 2003131853 | A1 | 17-07-2003 | NONE | | |
| US 2020171255 | A1 | 04-06-2020 | CN | 107320831 A | 07-11-2017 |
| | | | US | 2020171255 A1 | 04-06-2020 |
| | | | WO | 2019028936 A1 | 14-02-2019 |
| WO 8000538 | A1 | 03-04-1980 | AU | 535174 B2 | 08-03-1984 |
| | | | CA | 1129745 A | 17-08-1982 |
| | | | DE | 2953031 C1 | 19-08-1982 |
| | | | GB | 2039747 A | 20-08-1980 |
| | | | JP | S579828 B2 | 23-02-1982 |
| | | | JP | S55500673 A | 18-09-1980 |
| | | | US | 4198970 A | 22-04-1980 |
| | | | WO | 8000538 A1 | 03-04-1980 |

EPO FORM P0459

For more details about this annex : see Official Journal of the European Patent Office, No. 12/82